# EUROPEAN PATENT SPECIFICATION

(11) **EP 3 797 771 B1**
(45) Date of publication and mention of the grant of the patent: **23.02.2022**
(21) Application number: 19020503.9
(22) Date of filing: 03.09.2019
(51) Int. Cl.: A61K 31/4704, A61P 1/00, A61P 1/04

(54) **REBAMIPIDE FOR USE IN PROPHYLAXIS AND TREATMENT OF CELIAC DISEASE**
REBAMIPID ZUR VERWENDUNG IN DER PROPHYLAXE UND BEHANDLUNG VON ZÖLIAKIE
REBAMIPIDE DESTINÉ À ÊTRE UTILISÉ DANS LA PRÉVENTION ET LE TRAITEMENT DE LA MALADIE CÉLIAQUE

(43) Date of publication of application: 31.03.2021
(73) Proprietor: SQUARE POWER LTD, London EC2A 3DQ (GB)
(72) Inventor:

(56) References cited:
- T. MATYSIAK-BUDNIK ET AL: "Review article: rebamipide and the digestive epithelial barrier", ALIMENTARY PHARMACOLOGY & THERAPEUTICS., vol. 18, no. s1, July 2003 (2003-07), pages 55-62, XP055667986, GB ISSN: 0269-2813, DOI: 10.1046/j.1365-2036.18.s1.6.x
- TETSUO ARAKAWA ET AL: "15th Anniversary of Rebamipide: Looking Ahead to the New Mechanisms and New Applications", DIGESTIVE DISEASES AND SCIENCES, KLUWER ACADEMIC PUBLISHERS-PLENUM PUBLISHERS, NE, vol. 50, no. 1, October 2005 (2005-10), pages S3-S11, XP019237499, ISSN: 1573-2568, DOI: 10.1007/S10620-005-2800-9
- YUJI NAITO ET AL: "Rebamipide: a gastrointestinal protective drug with pleiotropic activities", EXPERT REVIEW OF GASTROENTEROLOGY & HEPATOLOGY ENGLAND, vol. 4, no. 3, June 2010 (2010-06), pages 261-270, XP055668022, DOI: 10.1586/egh.10.25
- GIACOMO CAIO ET AL: "Celiac disease: a comprehensive current review", BMC MEDICINE, vol. 17, no. 1, 23 July 2019 (2019-07-23), page 142, XP055667941, DOI: 10.1186/s12916-019-1380-z

## Description

### Field of the Invention

The present invention relates to rebamipide for use in prevention and/or treatment of celiac disease, in particular in patients suffering from increased intestinal permeability or in patients which are at risk of increased intestinal permeability. Furthermore, it relates to rebamipide for use in a method of inhibiting a gluten-induced increase in intestinal permeability.

### Background Art

Celiac disease (CD), also known as gluten-sensitive enteropathy, is a multifactorial autoimmune disorder that occurs in genetically susceptible individuals, especially in those expressing the HLA-DQ2/-DQ8 haplotype. It is caused by an intolerance to gluten, which is a protein naturally occurring in wheat, rye, barley, and related grains. If a person suffers from celiac disease, eating gluten triggers autoimmune inflammation of the intestinal mucosa. Over time, this reaction damages the villi, small fingerlike projections lining the small intestine, that promote nutrient absorption. When the villi get damaged, nutrients cannot be absorbed properly into the body. The intestinal damage often causes diarrhea, fatigue, weight loss, bloating and anemia, and can lead to serious complications.

The disorder may appear at any age and is estimated to affect 1 in 100 people worldwide. Children often develop symptoms shortly after being exposed to gluten for the first time, but symptoms may also appear after safely consuming gluten products for years. In the latter case the disorder may manifest after a triggering event such as illness, pregnancy or a stressful live event.

Gluten is a mixture of seed storage proteins, which comprise up to 80% of the protein contained in wheat seed. Dietary gluten is comprised of a number of homologous proteins called gliadins and glutenins in wheat, hordeins in barley, and secalins in rye. Each of these proteins harbors multiple disease-specific T-cell epitopes. The gluten proteins have similar amino acid sequences and often contain repeating stretches that are dominated by proline and glutamine residues. After gluten enters into the digestive system, it is partially hydrolyzed by proteases presented in the gastrointestinal tract. However, the high content of proline makes the gluten proteins particularly resistant to extensive proteolysis by intraluminal proteases and brush-border membrane enzymes of enterocytes, which results in long fragments of undigested gluten proteins surviving in the upper part of the small bowel, where they can become exposed to the inductive part of the gut immune system as immunogenic peptides permitting responses by T cells and B cells. Elimination of these proteins from the diet leads to complete remission.

The human intestine is lined up by a single layer of epithelial cells that are held together by tight junctions, adherens junctions and desmosomes. Peptides may cross the intestinal epithelium either via the paracellular route or via the transcellular route. Under normal physiological conditions, the majority (~90%) of antigens that pass through the intestinal epithelium travel through the transcellular pathway. The transcellular pathway is regulated and leads to lysosomal degradation of antigens into small non-immunogenic peptides. The remaining ~10% of proteins cross the epithelium through the paracellular pathway as full intact proteins or partially digested peptides as a tightly regulated antigen trafficking through intestinal tight junction modulation which leads to antigenic tolerance.

In healthy individuals, gluten peptides that are endocytosed by enterocytes are almost totally degraded by the lysosomal system during transcellular transport (Matysiak-Budnik T, Candalh C, Dugave C, Namane A, Cellier C, Cerf-Bensussan N, Heyman M: Alterations of the intestinal transport and processing of gliadin peptides in celiac disease. Gastroenterology 2003;125:696 -707), thus likely avoiding excessive activation of the local immune system. In contrast, in active CD, more peptides cross the intestinal mucosa and a significant fraction reach the lamina propria in intact form. The mechanism involves gluten peptides binding to the secretory IgA at the apical membrane of intestine, followed by binding the transferrin receptor CD71 on the enterocyte. This is then transcytosed, delivering the gluten peptides to the lamina propria (Matysiak-Budnik T et al. Secretory IgA mediates retrotranscytosis of intact gliadin peptides via the transferrin receptor in celiac disease. J Exp Med. 2008;205(1): 143-54).

Interestingly, gluten peptides pass the epithelial layer also through the paracellular pathway, which involves disassembling the intercellular tight junction. Upon reaching the intestinal lumen, gluten peptide fragments bind to chemokine C-X-C receptor 3 (CXCR3), thus recruiting myeloid differentiation primary-response protein 88 (MyD88), which induces the release of zonulin to the lumen (Lammers KM et al. Gliadin induces an increase in intestinal permeability and zonulin release by binding to the chemokine receptor CXCR3, Gastroenterology 2008;135(1):194-204). Zonulin is the only described physiological modulator of intercellular tight junctions. It binds to epidermal growth factor receptor (EGFR) and protease-activated receptor 2 (PAR2) in the intestinal epithelium. This complex initiates a signaling pathway that results in phosphorylation of zonula occludens proteins and leads to small intestine tight junction disassembling leading to increased intestinal permeability to macromolecules, thus enabling paracellular leakage of gluten peptides.

Similar to many other autoimmune diseases, CD involves innate and adaptive immune responses. At first, gluten peptides passage into the lamina propria by a mechanism described above. Here, they can activate both dendritic cells and macrophages to produce interleukin 15 (IL-15) which in turn up-regulates the expression of stress proteins MICA/B and HLA-E on intestinal enterocytes. These proteins are recognized by the natural killer receptors NKG2D and NKG2C present on intraepithelial lymphocytes (IELs), the expression of which is also up-regulated by IL-15 (Hüe S et al. A direct role for NKG2D/MICA interaction in villous atrophy during celiac disease. Immunity. 2004; 21(3):367-7; Kinoshita N et al. Autocrine IL-15 mediates intestinal epithelial cell death via the activation of neighboring intraepithelial NK cells. J Immunol. 2002; 169(11):6187-92). This process causes cytotoxicity and apoptosis-inducing activity of IELs and epithelial cell damage via perforin/granzyme and/or Fas/FasL pathways (Ciccocioppo R et al. Cytolytic mechanisms of intraepithelial lymphocytes in coeliac disease (CoD). Clin Exp Immunol. 2000;120:235-40).

The adaptive immune response involves the binding of gluten peptides present in the intestinal lamina propria to antigen-presenting cells (APCs), in particular to DCs. These cells present gluten peptides to T cell receptors, which leads to strong activation of CD4+ T cells able to induce lymphocyte B differentiation into plasma cells producing specific antibodies against gluten. Furthermore, gluten peptides undergo deamidation by tissue transglutaminase (tTG or TG2), which enhances their affinity to MHC II molecules, such as HLA-DQ2 or DQ8, displayed on APCs. This deamidation process enables the APCs to take up not only immunogenic peptides but also tTG-peptide complexes. As a consequence, antibodies are produced not only against gluten peptides but also against tTG (Di Sabatino A et al. The function of tissue transglutaminase in celiac disease. Autoimmun Rev. 2012;11(10):746-53). CD4+ T cells are also secreting pro-inflammatory cytokines, of which interferon (IFN)-γ is dominant, that stimulate cytotoxic T cells and fibroblasts to produce matrix metalloproteases (MMPs) responsible for degradation of both extracellular matrix and basement membrane. The above described inflammatory process leads to disruption of the structure and function of the small bowel's mucosal lining leading to villous flattening and crypt hyperplasia. Currently, the only known effective treatment for celiac disease is a strict lifelong gluten free diet, which leads to recovery of the intestinal mucosa, improves symptoms and reduces risk of developing complications in most people. If untreated, it may result in cancers such as intestinal lymphoma. Therefore, there is an increased desire for alternative therapies.

Rebamipide, which is chemically 2-[(4-chlorobenzoyl)amino]-3-(2-oxo-1H-quinolin-4-yl)propanoic acid) is used for the treatment of acute and/or chronic gastritis and gastroduodenal ulcers. Its mechanism of action relates to mucosal defense, scavenging free radicals, and temporarily activating genes encoding cyclooxygenase-2.

### Summary of the Invention

It has been unexpectedly found that rebamipide, when administered to patients suffering from celiac disease, is able to suppress the disease symptoms and may even lead to complete remission. This is very surprising since it hasn't been reported that rebamipide, which is clinically used for the treatment of gastric conditions, could be effective against celiac disease affecting primarily the small intestine. Although rebamipide has been marketed since 1990, little is known about its behavior in the intestine. The molecule is poorly absorbed in the GI tract, which indicates that following oral administration it may reach the intestinal mucosa in a concentration high enough to exert a therapeutic effect. From this point of view, rebamipide's presumed mechanism of action against the CD is likely based on the ability to induce mucine production in the intestine, to suppress inflammation and to reduce permeability of the intestinal wall.

The present invention thus provides rebamipide or a pharmaceutical composition thereof for use in a method of prevention and/or treatment of celiac disease, in particular by decreasing the intestinal permeability. Preferably, rebamipide is used in prevention and/or treatment of celiac disease in a person adhering to gluten-free diet.

"Rebamipide", as used herein, shall include all forms of this active ingredient, such as anhydrous form, hydrated or solvated form (e.g. hemihydrate form), crystalline forms; and pharmaceutically acceptable salts thereof.

"Prevention" or "preventive use" shall be understood herein as preventing or delaying the onset of the celiac disease including recurrence of its symptoms in patients with complete or partial remission, e.g. after gluten-free diet or any other previous treatment. It is also meant to include non-worsening of the disease symptoms after gluten transgression in a person otherwise adhering to gluten-free diet.

"Treatment" shall be understood herein as a therapy that is able to abrogate, inhibit, slow or reverse the progression of the celiac disease, and includes inhibition of chronic inflammation of the small intestine, reduction of villous atrophy and/or crypt hyperplasia, decrease in abnormal intestinal permeability and progressive disappearance of autoantibodies. It is also meant to cover amelioration or alleviation of clinical symptoms of the disease, such as diarrhea, bloating, fatigue, abdominal pain, nausea and vomiting, constipation, weight loss, mouth ulcers, iron-deficiency anemia, depression or anxiety, dermatitis herpetiformis, headache, loss of bone density, arthritis, etc.

"Gluten", as used herein, shall encompass proteins present in wheat or related grain species, including barley, rye and oat, which are potentially harmful to certain individuals. Gluten proteins particularly include alpha, beta, gamma and omega gliadins, and low and high molecular weight (LMW and HMW) glutenins in wheat; B, C and D hordeins in barley; beta, gamma and omega secalins in rye; and avenins in oats.

The current body of evidence suggests that multiple factors, such as genetic makeup and environmental triggers, play role in the development of the celiac disease. Increased intestinal permeability has been suggested to be another key element in the pathogenesis of the CD since it enables entry of gluten peptides into the lamina propria, where the gluten-reactive T-cells reside. Studies on Irish setter dogs, naturally occurring gluten sensitivity model, have shown that an increase in intestinal permeability may exist even prior to onset of this autoimmune disease in them (Hall EJ, Batt RM. Abnormal permeability precedes the development of a gluten sensitive enteropathy in Irish setter dogs. Gut. 1991;32;749-753). In the framework of the present invention, it was surprisingly found that rebamipide is also able to help normalize intestinal permeability in the CD patients. This is likely due to its ability to restore the function of the tight junctions of epithelial cells, thus reducing the permeability of the intestinal wall and preventing passage of gluten into the lamina propria. This contributes to suppression of chronic inflammation and prevents further tissue damage by autoimmune reactions.

In one aspect, the present invention provides rebamipide for use in a method of prevention and/or treatment of celiac disease in a person suffering from increased intestinal permeability or in a person which is at risk of increased intestinal permeability, e.g., due to family anamnesis or due to exposure to conditions or substances inducing increased intestinal permeability. In a preferred embodiment rebamipide is for use in a method of prevention of the celiac disease.

"Increased intestinal permeability" is used herein as a term designating little intestinal wall defects, including those caused by subclinical chronic inflammation (low grade inflammation) of the gut wall. These intestinal wall defects may be manifested by the celiac disease but also by other medical conditions, such as chronic constipation or gastroparesis. Increased intestinal permeability may be diagnosed using specific tests, such as lactulose-mannitol test (La/Ma test; e.g. Uil JJ et al. Sensitivity of a hyperosmolar or low-osmolar test solution for sugar absorption in recognizing small intestinal mucosal damage in coeliac disease. Digest Liver Dis 2000; 32:195-200), A-1-AT test, or zonulin test. Typically, increased intestinal permeability is permeability of the intestinal wall to particles having the size of more than 4 Angstroms in radius.

Substances inducing increased intestinal permeability include non-steroidal anti-inflammatory drugs (NSAIDs), such as acetylsalicylic acid, ibuprofen, naproxen, ketoprofen, fenoprofen, flurbiprofen, diclofenac, ketorolac, etodolac, indomethacin, tolmetin, piroxicam, meloxicam and selective COX-2 inhibitors such as celecoxib and etoricoxib; alcohol; nicotine; food additives; antibiotics; and chemotherapeutics. Thus, simultaneous or sequential coadministration of rebamipide with non-steroidal anti-inflammatory drugs, chemotherapeutics or antibiotics prevents intestinal wall damage and thus prevents or delays the onset of the celiac disease associated with increased intestinal permeability. Prophylactic use of rebamipide may also be useful in persons abusing alcohol, nicotine or other drugs known to damage intestinal wall. The term "abuse" as used herein is meant to include any consumption, which is not necessary for medical reasons and leads to dependency and/or health impairments including low grade inflammation of the gut wall.

Conditions inducing increased intestinal permeability are related to stress, imbalanced diet, allergy, bacterial, viral or parasitic infections and various medical treatments. Such conditions in particular include stress-induced gastritis, alimentary intoxication, disbalance of cholic acids, gastric HCl and pepsin secretion, non-infectious diarrhea, radiation therapy, chemotherapy, infectious or post-infectious impairment of the GIT mucosa, dysmicrobia (e.g. induced by antibiotic treatment).

The persons suffering from increased intestinal permeability typically suffer from at least one condition selected from low grade inflammation of the gut wall, chronic constipation or gastroparesis.

In one aspect, the present invention provides rebamipide for use in a method of inhibiting a gluten-induced increase in intestinal permeability. The method comprises administering rebamipide or a composition thereof to a subject in need thereof, in an amount sufficient to inhibit said increase in intestinal permeability.

In the therapeutic indications as described in the present invention, rebamipide may preferably be used in oral pharmaceutical forms such as tablets, capsules, dragees, granules, microgranules (sachets), orodispersible tablets or films, sublingual tablets, crushed tablets, oral solutions, oral suspensions, syrups, mouthwashes or rinses. Alternatively, it can be used in rectal pharmaceutical forms such as suppositories and enemas. Preferably, the oral pharmaceutical forms, such as tablets, capsules, dragees and granules, is a form with enteric release, such as enteric sustained release or enteric controlled release.

The pharmaceutical forms may contain at least one pharmaceutically acceptable excipient selected from fillers, binders, lubricants, glidants, disintegrants/swelling agents, solubilizers, enteric release agents, mucoadhesive components, sustained release agents, preservatives, coatings and colorants. Such excipients are known in the art of pharmaceutical formulation, and the skilled person is capable of selecting suitable excipients for the relevant pharmaceutical forms.

Suitable methods for preparing the pharmaceutical forms and compositions includes the processes of wet granulation or dry granulation of the active ingredient with the auxiliary substances and components, or direct homogenization of the active ingredient with the auxiliary substances and components.

Fillers may preferably be selected from saccharide alcohols (such as mannitol, sorbitol, xylitol), lactose, starch, pregelatinized starch, cellulose, sillicified cellulose, calcium hydrogen phosphate, calcium phosphate, sucrose and calcium sulphate. The fillers may preferably be present in the amount of 5 to 90 wt. %, relative to the total weight of the composition.

Binders may preferably be selected from starch, pregelatinized starch, povidone, copovidone, hydroxypropyl methylcellulose, hydroxypropyl cellulose, ethyl cellulose, cellulose. The binders may preferably be present in the amount of 1 to 20 wt. %, relative to the total weight of the composition.

Lubricants may preferably be selected from magnesium stearate, calcium stearate, stearic acid, polyethylene glycol and sodium stearyl fumarate. The lubricants may preferably be present in the amount up to 5 wt. %, relative to the total weight of the composition.

Glidants may preferably be selected from silica, talc and sodium lauryl sulphate. The glidants may preferably be present in the amount of 0.5 to 10 wt. %, relative to the total weight of the composition.

Swelling and/or disintegrating agents may preferably be selected from crospovidone, copovidone, povidone, croscarmellose, hydroxypropyl methylcellulose, starch, pregelatinized starch, low-substituted hydroxypropyl cellulose, sodium carboxymethyl starch. The swelling / disintegrating agents may preferably be present in the amount of 1 to 50 wt. %, relative to the total weight of the composition.

Solubilizers may preferably be selected from poloxamer, sodium lauryl sulphate, polysorbate, polyoxylated oleic glycerides, glycerol monostearate and cyclodextrins. The solubilizers may preferably be present in the amount up to 30 wt. %, relative to the total weight of the composition.

Enteric release agents may preferably be selected from hydroxypropyl methylcellulose phthalate, poly(methacrylic acid-co-methyl methacrylate), cellulose acetate phthalate, poly(vinyl acetate phthalate), esters of aleuritic acid. The enteric release agents may preferably be present in the amount of 2 to 40 wt. %, relative to the total weight of the composition.

Mucoadhesive components may preferably be selected from propylene glycol alginate, sodium alginate, calcium alginate, potassium alginate, hydroxypropyl methylcellulose, sodium carmellose, polyacrylic acid, polyethylene oxide, povidone and copovidone. The mucoadhesive components may preferably be present in the amount of 5 to 70 wt. %, relative to the total weight of the composition.

Sustained release agents may preferably be selected from cellulose and cellulose ethers such as hydroxypropyl cellulose, hydroxypropyl methylcellulose, carboxymethylcellulose, sodium carboxymethylcellulose, ethyl cellulose, methylcellulose, polyvinyl acetate, alginic acid, propylene glycol alginate, sodium alginate, calcium alginate, potassium alginate, polymethacrylates, guar gum, xanthan gum, carrageenan, castor oil, beeswax, carnauba wax, glycerol palmitostearate, glycerol monostearate, glycerol behenate, stearyl alcohol, polyacrylic acid. The sustained release agents may preferably be present in the amount of 5 to 70 wt. %, relative to the total weight of the composition.

The oral pharmaceutical composition may in some embodiments further contain a pharmaceutically acceptable component capable of forming carbon dioxide upon contact with gastric juices, such component may preferably be selected from carbonates and hydrogen carbonates of alkali metals and alkaline earth metals; and may preferably be present in an amount in the range from 1 to 50 wt. %, relative to the total weight of the composition.

A typical daily dose of rebamipide may range from 1 to 5000 mg for an average human (70 kg weight), more preferably from 50 to 2500 mg, even more preferably from 100 to 1000 mg, and most preferably from 300 to 500 mg.

When immediate release formulation of rebamipide is administered, the daily dose is typically divided into several doses, which are administered separately. The daily dose may be divided into two to six separate doses taken twice daily or three times per day or four times per day or five times per day or six times per day. In a preferred embodiment the daily dose is divided into three separate doses administered three times per day, especially 100 mg dose administered three times per day. Alternatively, the whole daily dose can be taken at once, especially if it is in the form of a sustained release formulation, e.g. 300 mg dose administered once daily. The duration of the treatment is to be determined by a physician.

Hereinafter, the present invention will be described more specifically by the following working example. However, the following working example is provided only for illustration and thus the present invention is not limited to it.

### Example

### Elimination-provocation test

To study the effect of rebamipide on the celiac disease, a gluten challenge test was employed. Adult patients admitted into the test were stabilized on a gluten-free diet (GFD) leading to disappearance of the disease symptoms for at least 12 months. All participants were previously diagnosed by a physician for the celiac disease using upper endoscopy and duodenal biopsy. The gluten-free diet adherence was confirmed by high villous height/crypt depth ratio (Vh/Cd > 2-3) and decline in anti-tTG antibody levels (less than 10 U/mL) after the exclusion of gluten from the diet.

Patients were divided into two groups taking either rebamipide 100 mg three times a day or placebo just before each major meal. Both groups continued the gluten-free diet. The gluten challenge test was carried out three month (day 90) after the start of the treatment. Patients were given one slice of white bread comprising about 2.7 g of wheat gluten every day for the period of 6 weeks but otherwise maintained their usual gluten-free diet. Both groups continued receiving either rebamipide or placebo treatment.

CD symptoms were weekly evaluated by the Gastrointestinal Symptom Rating Scale (GSRS) questionnaire, an instrument that is widely used in research on celiac disease and other gastrointestinal disorders (Svedlund J, Sjodin I, Dotevall G. GSRS-a clinical rating scale for gastrointestinal symptoms in patients with irritable bowel syndrome and peptic ulcer disease. Dig Dis Sci 1988;33:129-134). It consists of 15 questions that are grouped into five domains (diarrhea, abdominal pain, indigestion, constipation, and reflux), which are combined into a total score calculated as a mean of all 15 questions. The scoring is based on a Likert scale from 1 to 7 points, where 1 point indicates minimal gastrointestinal symptoms and 7 points the most severe symptoms.

The intestinal permeability was measured using the standard urinary lactulose/mannitol (La/Ma) fractional excretion ratio at the beginning of the test, before and after 6-week challenge. The La/Ma test is based on a different absorption of non-metabolized sugars. In the healthy intestine, monosaccharides (e.g. mannitol) are passively absorbed through the intestinal mucosa, while larger disaccharide molecules (e.g. lactulose) are normally almost not absorbed. On the contrary, patients with the celiac disease have a consistent increase in the absorption of lactulose (via the paracellular route) due to increased permeability of the intestine and a reduction in the absorption of mannitol (via the transcellular route) due to a reduction in surface area as a result of villous atrophy, resulting in an increase in the La/Ma ratio.

Duodenal biopsy was performed at the beginning (day 0) and at the end (day 132) of the study period. Villous height to crypt depth ratio (Vh/Cd) was determined by measuring the mean height/mean depth of adjacent villi/crypts by a pathologist. The ratio of < 2 is regarded as indicative of villous atrophy and crypt hyperplasia.

Serum mean anti-tTG IgA antibody levels were determined by an enzyme-linked immunosorbent assay (ELISA) and the difference at baseline and after 6-week challenge was compared.

### Results are summarized in Table 1

**Table 1**

| | Rebamipide Group | | | Placebo Group | | |
|---|---|---|---|---|---|---|
| | day 0 | day 90 | day 132 | day 0 | day 90 | day 132 |
| GSRS | 2.0 | 1.7 | 1.8 | 1.9 | 1.8 | 2.4 |
| La/Ma | 0.057 | 0.038 | 0.048 | 0.064 | 0.051 | 0.168 |
| Vh/Cd | 2.9 | N/A | 3.1 | 3.0 | N/A | 1.7 |
| anti-tTG IgA | 3.9 | 4.3 | 7.8 | 4.8 | 5.5 | 35.6 |

The mean total GSRS score decreased during the rebamipide pretreatment followed by gradual increase toward the baseline values over the course of the gluten challenge. In contrast, the placebo group experienced a rapid increase in the GSRS score during the first week of the gluten challenge and it remained elevated thereafter. The patients reported a wide range of symptoms including diarrhea, bloating, abdominal pain, and tiredness. The difference in GSRS scores for rebamipide versus placebo showed that the rebamipide treated group had the mean GSRS scores significantly lower than the placebo group (0.6 units at the end of the study). This means that the symptoms were statistically significantly less severe in the group consisting of patients who were receiving rebamipide before and during the gluten challenge compared with the placebo group.

Although the La/Ma fractional excretion is known to be highly variable from person-to-person, the results show that the ratio of these sugars significantly increased after daily gluten intake in the placebo group suggesting an increase in intestinal permeability. In contrast, the La/Ma ratio in the rebamipide treated group remained near the baseline at the end of the gluten challenge, indicating its activity against undesirably increased intestinal permeability.

Beginning on day 0, all participants had normal Vh/Cd ratios on small-intestinal biopsy. However, histological changes were observed in the majority of patients from the placebo group after 6 weeks of daily gluten intake. The mean Vh/Cd ratio worsened to 1.7, indicating active celiac disease. Surprisingly, patients in the rebamipide group didn't exhibit negative changes from baseline to post treatment in villus height to crypt depth ratio, suggesting a successful treatment.

The mean anti-tTG IgA antibody titers were increased after the gluten challenge but remained below the 10 U/ml threshold in rebamipide treated group. The placebo group showed stronger mean antibody response, although some patients didn't develop any measurable change in the level of anti-tTG IgA over the course of the gluten challenge.

The results of the elimination-provocation test show that rebamipide provides protection of the intestinal mucosa against ingested gluten, decreases the intestinal permeability and improves patient reported symptoms of the celiac disease. The drug allows gluten to be consumed without the disease exacerbation, which may dramatically improve the quality of life since adherence to a strict gluten-free diet is difficult and costly. Moreover, accidental gluten ingestion cannot be completely avoided and traces of gluten can be found even in gluten-free-labeled products. It can be concluded that rebamipide offers a unique opportunity in the celiac disease management, improves patients' quality of life and may secure a long-term remission of the disease.

## Claims

1. Rebamipide for use in a method of prevention and/or treatment of celiac disease.

2. Rebamipide for use according to claim 1, wherein rebamipide is used in prevention and/or treatment of celiac disease in a person adhering to gluten-free diet.

3. Rebamipide for use in a method of inhibiting gluten-induced increase in intestinal permeability.

4. Rebamipide for use according to claim 1, wherein rebamipide is used in prevention and/or treatment of celiac disease in a person suffering from increased intestinal permeability or in a person which is at risk of increased intestinal permeability.

5. Rebamipide for use according to any one of the preceding claims, wherein rebamipide is used in a method of prevention of celiac disease.

6. Rebamipide for use according to claim 4 or 5, wherein the person suffering from increased intestinal permeability is a person suffering from at least one condition selected from low grade inflammation of the gut wall, chronic constipation or gastroparesis.

7. Rebamipide for use according to claim 4 or 5, wherein the person at risk of increased intestinal permeability is a person suffering from stress, imbalanced diet, bacterial, viral or parasitic infection.

8. Rebamipide for use according to claim 4 or 5, wherein the person at risk of increased intestinal permeability is a person exposed to at least one substance selected from non-steroidal anti-inflammatory drugs, alcohol, nicotine, food additives, chemotherapeutics and antibiotics.

9. Rebamipide for use according to claim 8, wherein rebamipide is co-administered simultaneously or sequentially with non-steroidal anti-inflammatory drug, chemotherapeutic or antibiotic.

10. Rebamipide for use according to claim 8, wherein rebamipide is administered to a person abusing alcohol, nicotine or another drug.

11. Rebamipide for use according to claim 4 or 5, wherein the person at risk of increased intestinal permeability is a person suffering from or exposed to at least one condition selected from stress-induced gastritis, alimentary intoxication, disbalance of cholic acids, gastric HCl and pepsin secretion, non-infectious diarrhea, radiation therapy, chemotherapy, infectious or post-infectious impairment of the GIT mucosa, dysmicrobia.

12. Rebamipide for use according to any one of the preceding claims, wherein rebamipide is administered in an oral pharmaceutical form, preferably selected from tablets, capsules, dragees, granules, microgranules (sachets), orodispersible tablets or films, sublingual tablets, crushed tablets, oral solutions, oral suspensions, syrups, mouthwashes, rinses; or in a rectal pharmaceutical form, preferably selected from suppositories and enemas.

13. Rebamipide for use according to claim 12, wherein the oral pharmaceutical form is a form with enteric release, preferably enteric sustained release or enteric controlled release.

14. Rebamipide for use according to claim 13, wherein the pharmaceutical form contains rebamipide and at least one pharmaceutically acceptable excipient selected from fillers, binders, lubricants, glidants, disintegrants/swelling agents, solubilizers, enteric release agents, mucoadhesive components, sustained release agents, preservatives, coatings and colorants.

15. Rebamipide for use according to any one of the preceding claims, wherein rebamipide is administered in a daily dose of 1 to 5000 mg, more preferably from 50 to 2500 mg, even more preferably from 100 to 1000 mg, and most preferably from 300 to 500 mg.

## Patentansprüche

1. Rebamipid zur Verwendung in einem Verfahren zur Prävention und/oder Behandlung der Zöliakie.

2. Rebamipid zur Verwendung nach Anspruch 1, **dadurch gekennzeichnet, dass** Rebamipid zur Prävention und/oder Behandlung der Zöliakie bei einer Person verwendet wird, die eine glutenfreie Diät einhält.

3. Rebamipid zur Verwendung in einem Verfahren zur Hemmung der gluteninduzierten Erhöhung der Darmpermeabilität.

4. Rebamipid zur Verwendung nach Anspruch 1, **dadurch gekennzeichnet, dass** Rebamipid zur Prävention und/oder Behandlung von Zöliakie bei einer Person verwendet wird, die an erhöhter Darmpermeabilität leidet oder bei einer Person, bei der das Risiko einer erhöhten Darmpermeabilität besteht.

5. Rebamipid zur Verwendung nach einem der vorangehenden Ansprüche, **dadurch gekennzeichnet, dass** Rebamipid in einem Verfahren zur Prävention von Zöliakie verwendet wird.

6. Rebamipid zur Verwendung nach Anspruch 4 oder 5, **dadurch gekennzeichnet, dass** die an erhöhter Darmpermeabilität leidende Person eine Person ist, die an mindestens einem Zustand leidet, ausgewählt aus einer niedriggradigen Entzündung der Darmwand, chronischer Verstopfung oder Gastroparese.

7. Rebamipid zur Verwendung nach Anspruch 4 oder 5, **dadurch gekennzeichnet, dass** die Person mit dem Risiko einer erhöhten Darmpermeabilität eine Person ist, die an Stress, unausgewogener Ernährung, bakterieller, viraler oder parasitärer Infektion leidet.

8. Rebamipid zur Verwendung nach Anspruch 4 oder 5, **dadurch gekennzeichnet, dass** die Person mit dem Risiko einer erhöhten Darmpermeabilität eine Person ist, die mindestens einer Substanz ausgesetzt ist, ausgewählt aus nicht-steroidalen entzündungshemmenden Arzneimitteln, Alkohol, Nikotin, Nahrungsergänzungsmitteln, Chemotherapeutika und Antibiotika.

9. Rebamipid zur Verwendung nach Anspruch 8, **dadurch gekennzeichnet, dass** Rebamipid gleichzeitig oder nacheinander mit einem nicht-steroidalen entzündungshemmenden Arzneimittel, Chemotherapeutikum oder Antibiotikum verabreicht wird.

10. Rebamipid zur Verwendung nach Anspruch 8, **dadurch gekennzeichnet, dass** Rebamipid an eine Person verabreicht wird, die Alkohol, Nikotin oder eine andere Droge missbraucht.

11. Rebamipid zur Verwendung nach Anspruch 4 oder 5, **dadurch gekennzeichnet, dass** die Person mit dem Risiko einer erhöhten Darmpermeabilität eine Person ist, die an mindestens einem Zustand leidet oder diesem ausgesetzt ist, ausgewählt aus stressinduzierter Gastritis, alimentärer Intoxikation, Ungleichgewicht von Cholsäuren, Magen-HCl und Pepsin Sekretion, nichtinfektiöser Durchfall, Strahlentherapie, Chemotherapie, infektiöse oder postinfektiöse Beeinträchtigung der GIT schleimhaut, Dysmikrobien.

12. Rebamipid zur Verwendung nach einem der vorangehenden Ansprüche, **dadurch gekennzeichnet, dass** Rebamipid in einer oralen pharmazeutischen Form verabreicht wird, vorzugsweise ausgewählt aus Tabletten, Kapseln, Dragees, Granulat, Mikrogranulat (Sachets), Schmelztabletten oder -filmen, Sublingualtabletten, zerstoßenen Tabletten, oral Lösungen, Suspensionen zum Einnehmen, Sirupe, Mundwässer, Spülungen; oder in einer rektalen pharmazeutischen Form, vorzugsweise ausgewählt aus Zäpfchen und Einläufen.

13. Rebamipid zur Verwendung nach Anspruch 12, **dadurch gekennzeichnet, dass** die orale pharmazeutische Form eine Form mit enterischer Freisetzung ist, vorzugsweise enterische verzögerte Freisetzung oder enterische kontrollierte Freisetzung.

14. Rebamipid zur Verwendung nach Anspruch 13, **dadurch gekennzeichnet, dass** die pharmazeutische Form Rebamipid und mindestens einen pharmazeutisch verträglichen Hilfsstoff enthält, ausgewählt aus Füllstoffen, Bindemitteln, Gleitmitteln, Glidantien, Sprengmitteln/Quellmitteln, Lösungsvermittlern, magensaftresistenten Wirkstoffen, mucoadhäsiven Komponenten, Wirkstoffen mit verzögerter Wirkstofffreisetzung, Konservierungsmitteln , Beschichtungen und Farbstoffe.

15. Rebamipid zur Verwendung nach einem der vorangehenden Ansprüche, **dadurch gekennzeichnet, dass** Rebamipid in einer Tagesdosis von 1 bis 5000 mg, bevorzugter von 50 bis 2500 mg, noch bevorzugter von 100 bis 1000 mg und am meisten bevorzugt von 300 bis 500 mg verabreicht wird mg.

## Revendications

1. Rébamipide pour une utilisation dans une méthode de prévention et/ou de traitement de la maladie cœliaque.

2. Rébamipide pour une utilisation selon la revendication 1, **caractérisé en ce que** le rébamipide est utilisé dans la prévention et/ou le traitement de la maladie cœliaque chez une personne adhérant à un régime sans gluten.

3. Rebamipide pour une utilisation dans une méthode d'inhibition de l'augmentation de la perméabilité intestinale induite par le gluten.

4. Rébamipide pour une utilisation selon la revendication 1, **caractérisé en ce que** le rébamipide est utilisé dans la prévention et/ou le traitement de la maladie cœliaque chez une personne souffrant d'une perméabilité intestinale accrue ou chez une personne qui présente un risque d'augmentation de la perméabilité intestinale.

5. Rébamipide pour une utilisation selon l'une quelconque des revendications précédentes, **caractérisé en ce que** le rébamipide est utilisé dans une méthode de prévention de la maladie cœliaque.

6. Rébamipide pour une utilisation selon la revendication 4 ou 5, **caractérisé en ce que** la personne souffrant d'une perméabilité intestinale accrue est une personne souffrant d'au moins une condition choisie parmi une inflammation de bas grade de la paroi intestinale, une constipation chronique ou une gastroparésie.

7. Rébamipide pour une utilisation selon la revendication 4 ou 5 **caractérisé en ce que** la personne à risque d'augmentation de la perméabilité intestinale est une personne souffrant de stress, de régime alimentaire déséquilibré, d'infection bactérienne, virale ou parasitaire.

8. Rebamipide pour une utilisation selon la revendication 4 ou 5, **caractérisé en ce que** la personne à risque d'augmentation de la perméabilité intestinale est une personne exposée à au moins une substance choisie parmi les médicaments anti-inflammatoires non stéroïdiens, l'alcool, la nicotine, les additifs alimentaires, les agents chimiothérapeutiques et les antibiotiques.

9. Rébamipide pour une utilisation selon la revendication 8, **caractérisé en ce que** le rébamipide est co-administré simultanément ou séquentiellement avec un médicament anti-inflammatoire non stéroïdien, chimiothérapeutique ou antibiotique.

10. Rébamipide pour une utilisation selon la revendication 8, **caractérisé en ce que** le rébamipide est administré à une personne abusant de l'alcool, de la nicotine ou d'une autre drogue.

11. Rebamipide pour une utilisation selon la revendication 4 ou 5, **caractérisé en ce que** la personne à risque d'augmentation de la perméabilité intestinale est une personne souffrant ou exposée à au moins une condition choisie parmi la gastrite induite par le stress, l'intoxication alimentaire, le déséquilibre des acides choliques, la sécrétion gastrique de HCl et de pepsine, diarrhée non infectieuse, la radiothérapie, la chimiothérapie, atteinte infectieuse ou post-infectieuse de la muqueuse de TGI, la dysmicrobie.

12. Rébamipide pour une utilisation selon l'une quelconque des revendications précédentes, **caractérisé en ce que** le rébamipide est administré sous une forme pharmaceutique orale, de préférence choisie parmi les comprimés, gélules, dragées, granulés, microgranules (sachets), comprimés ou films orodispersibles, comprimés sublinguaux, comprimés écrasés, solutions, suspensions buvables, sirops, bains de bouche, rince-bouche; ou sous une forme pharmaceutique rectale, de préférence choisie parmi les suppositoires et les lavements.

13. Rébamipide pour une utilisation selon la revendication 12, **caractérisé en ce que** la forme pharmaceutique orale est une forme à libération entérique, de préférence à libération entérique prolongée ou à libération entérique contrôlée.

14. Rébamipide pour une utilisation selon la revendication 13, **caractérisé en ce que** la forme pharmaceutique contient du rébamipide et au moins un excipient pharmaceutiquement acceptable choisi parmi les charges, les liants, les lubrifiants, les glissants, les délitants/agents gonflants, les solubilisants, les agents à libération entérique, les composants mucoadhésifs, les agents à libération prolongée, les conservateurs, enduits et colorants.

15. Rébamipide pour une utilisation selon l'une quelconque des revendications précédentes, **caractérisé en ce que** le rébamipide est administré à une dose quotidienne de 1 à 5000 mg, plus préférablement de 50 à 2500 mg, encore plus préférablement de 100 à 1000 mg, et le plus préférablement de 300 à 500 mg.
